# EUROPEAN PATENT SPECIFICATION

(11) **EP 2 134 322 B1**
(45) Date of publication and mention of the grant of the patent: **20.02.2019**
(21) Application number: 08732162.6
(22) Date of filing: 13.03.2008
(51) Int. Cl.: A61K 9/19, A61K 31/404, A61P 35/00

(54) **INTRAVESICAL APAZIQUONE ADMINISTRATION FOLLOWING TRANSURETHRAL RESECTION FOR TREATING BLADDER CANCER**
INTRAVESIKALE VERABREICHUNG VON APAZICHON NACH TRANSURETHRALER RESEKTION ZUR BEHANDLUNG VON BLASENKREBS
ADMINISTRATION INTRAVÉSICALE D'APAZIQUONE APRÈS RÉSECTION TRANSURÉTHRALE POUR TRAITER LE CANCER DE LA VESSIE

(30) Priority: 13.03.2007 US 894610 P
(43) Date of publication of application: 23.12.2009
(73) Proprietor: Spectrum Pharmaceuticals, Inc., Irvine, CA 92618 (US)
(72) Inventor: LENAZ, Luigi, Newton, PA 18940 (US); CHAWLA, Shanta, Irvine, CA 92602 (US); BEER, Mario, San Clemente, CA 92672 (US)
(74) Representative: Potter Clarkson
(86) International application number: PCT/US2008/056912
(87) International publication number: WO 2008/112934

(56) References cited:
- US-B2- 6 894 071
- VAN DER HEIJDEN ET AL: "Phase II Marker Lesion Study With Intravesical Instillation of Apaziquone for Superficial Bladder Cancer: Toxicity and Marker Response" JOURNAL OF UROLOGY, BALTIMORE, MD, US, vol. 176, no. 4, 1 October 2006 (2006-10-01), pages 1349-1353, XP005657772 ISSN: 0022-5347
- PURI ET AL: "Phase I/II Pilot Study of Intravesical Apaziquone (EO9) for Superficial Bladder Cancer" JOURNAL OF UROLOGY, BALTIMORE, MD, US, vol. 176, no. 4, 1 October 2006 (2006-10-01), pages 1344-1348, XP005657771 ISSN: 0022-5347
- SYLVESTER ET AL: "A SINGLE IMMEDIATE POSTOPERATIVE INSTILLATION OF CHEMOTHERAPY DECREASES THE RISK OF RECURRENCE IN PATIENTS WITH STAGE Ta T1 BLADDER CANCER: A META-ANALYSIS OF PUBLISHED RESULTS OF RANDOMIZED CLINICAL TRIALS" JOURNAL OF UROLOGY, BALTIMORE, MD, US, vol. 171, no. 6, 1 June 2004 (2004-06-01), pages 2186-2190, XP005532462 ISSN: 0022-5347
- DRAGAN J GOLIJANIN ET AL: "Chemoprevention of bladder cancer" WORLD JOURNAL OF UROLOGY, SPRINGER-VERLAG, BE, vol. 24, no. 5, 18 October 2006 (2006-10-18), pages 445-472, XP019459697 ISSN: 1433-8726
- LOADMAN P M ET AL: "Pharmacological approach towards the development of indolequinone bioreductive drugs based on the clinically inactive agent EO9.", BRITISH JOURNAL OF PHARMACOLOGY, vol. 137, no. 5, November 2002 (2002-11), pages 701-709, ISSN: 0007-1188

## Description

### CROSS REFERENCE TO RELATED APPLICATIONS

This application claims the benefit of U.S. Provisional Patent Application No. 60/894,610, filed March 13, 2007.

### FIELD OF THE INVENTION

The methods described herein relate to treatments for bladder cancers. More specifically, the described methods relate to the intravesical administration of apaziquone following transurethral resection.

### BACKGROUND OF THE INVENTION

Bladder cancer is the seventh most common cancer worldwide. In 2006, there were an estimated 280,000 cases of bladder cancer in Europe and more than 60,000 new cases were expected in the United States. The most common type of bladder cancer (90%) is transitional cell carcinoma (TCC) which derives from the urothelium, the cellular lining of the urethral system (ureters, bladder and urethra). About 75% of newly detected bladder cancers are superficial at initial presentation, meaning that their entirety remains near the surface of the urothelium. More specifically, superficial tumors consist of papillary tumors that are confined to the mucosa (Ta), papillary or sessile tumors extending into the lamina propria (T1) and carcinoma *in situ* (CIS). All types are limited to the mucosal or submucosal layer without muscle invasion.

Superficial bladder cancers can be stratified into prognostic risk classes according to tumor stage, grade, size, number and recurrence pattern. Low-stage, low-grade primary tumors (stage Ta, grades G1-G2) have a 30% recurrence rate over 2 years and do not usually progress to muscle invasion, while at the other extreme, multiple, highly recurrent or large T1 G3 tumors have up to a 70%-80% recurrence rate and a 10%-30% progression rate to a muscle-invasive stage. Carcinoma *in situ* (CIS) presents the highest risk of tumor progression.

Initial treatment for papillary TCC presenting at stages Ta and T1 is usually complete transurethral resection of bladder tumor (TUR-BT) (i.e. surgical removal) of all visible lesions, while the presence of CIS generally indicates other additional measures due to its high risk of progression. For example, CIS patients are often given adjuvant treatments with intravesical instillation of a cytotoxic or immunologic agent (commonly Bacillus Calmette-Guerin (BCG)). BCG treatments have consistently shown an advantage over TUR-BT alone in terms of relapse-free survival. Adjuvant treatment with BCG can also lower the rate of progression to invasive disease.

Even though the risk of progression to an invasive stage is low in low-risk tumors, the multiple recurrence patterns of these superficial bladder cancers often requires repeated interventions and causes considerable trouble to the patient. Therefore, more effective treatments that reduce the recurrence of superficial bladder cancers would provide an important advance in the treatment of these tumors.

Van der Heijden et al., Journal of Urology, 176(4), 2006, 1349-1353 describes a study of the ablative activity of intravesical apaziquone on a papillary marker tumour in which the study determined the incidence of side effects.
Puri et al., Journal of Urology 176(4), 2006 1344-1348 is concerned with a study to determine the dose of apaziquone that can be safely administered intravesically and the study explored apaziquone's activity for superficial bladder transitional cell carcinoma.

### SUMMARY OF THE INVENTION

According to the present invention there is provided use of apaziquone (EO9) in the manufacture of a medicament for treating bladder cancer comprising administering via intravesical installation a therapeutic composition comprising a therapeutic amount of apaziquone (EO9), wherein said administration occurs within about 6 hours following transurethral resection of bladder tumor (TUR-BT). There is also provided apaziquone (EO9) for use in treating bladder cancer in accordance with claim 7. The invention further provides the use of E09 in the manufacture of a medicament for treating bladder cancers according to claim 5 and E09 for use in treating bladder cancers according to claim 11.

In accordance with the present invention the therapeutic composition is administered within about 6 hours following TUR-BT. In another aspect, the therapeutic composition is administered within about 5 hours following TUR-BT. In another aspect, the therapeutic composition is administered within about 4 hours following TUR-BT. In another aspect, the therapeutic composition is administered within about 3 hours following TUR-BT. In another aspect, the therapeutic composition may
be administered in a single dose. In another aspect, the therapeutic composition may be reconstituted and/or lyophilized before or after performing TUR-BT in patients in need thereof.

In accordance with the present invention, the cancer to be treated is bladder cancer. In another embodiment, the cancer to be treated is non-invasive bladder cancer (SBCs). In another embodiment the cancer to be treated is transitional-cell carcinoma of the bladder. In another embodiment, the cancer is TNM stage Ta or T1 and histologic grade G1 or G2 before TUR.

In one embodiment, the therapeutic composition comprises from about 1 mg to about 8 mg per dose apaziquone in a reconstituted lyophilized therapeutic composition. In another embodiment, the therapeutic compositions comprises from about 2 mg to about 6 mg per dose apaziquone. Optionally about 10 mg to about 200 mg mannitol is present, and optionally about 2 mg to about 300 mg sodium bicarbonate is present in a therapeutic composition. Also described herein the therapeutic composition comprises from about 0 ml_ to about 24 ml_ propylene glycol, and about 0 mg to about 10 mg EDTA. These therapeutic compositions may be administered to a patient in need of treatment for bladder cancer
following TUR-BT. In another embodiment, the lyophilized reconstituted therapeutic composition may be administered via a single intravesical instillation.

In one aspect, is a step which includes administering a volume of reconstituted lyophilized therapeutic composition of between about 2 ml_ and about 80 ml. In another aspect, the volume of reconstituted lyophilized therapeutic composition is
between about 30 mL and about 60 mL. In another aspect, the volume of reconstituted lyophilized therapeutic composition of about 40 mL.

In one embodiment, the therapeutic composition administered following TUR-BT may be prepared using diluents having about 0% to about 60% vol/vol propylene glycol, about 0 mg/mL to about 5 mg/mL EDTA, and about 0 mg/mL to about 20 mg/mL sodium bicarbonate, and water. In another embodiment, the diluents may have about 20% to about 40% vol/vol propylene glycol, about 0.01 mg/mL to about 1 mg/mL EDTA, and about 1 mg/mL to about 10 mg/mL sodium bicarbonate, and water. In another embodiment, the diluents may have about 30% (vol/vol) propylene glycol, about 0.1 mg/mL EDTA, about 5 mg/mL sodium bicarbonate, and water. In another aspect, the therapeutic composition may be reconstituted and/or lyophilized before or after performing TUR-BT in patients in need thereof. In another aspect, the therapeutic composition may be administered in a single instillation given within six hours of TUR-BT.

### DETAILED DESCRIPTION

Indoloquinone compounds are known to be useful as cytostatic agents for treating cancer in humans. See, for example, United States Patent Number 5,079,257 for all it teaches related to indoloquinone synthesis, metabolism and therapeutic activity. In addition, see for example, United States patent number 6,894,071 for all it teaches related to apaziquone formulations.

The present disclosure describes the use of preparations comprising the bioredutive alkylating indoloquinone with anti-tumor effects, apaziquone (EO9), following transurethral resection of bladder tumor (TUR-BT) for the treatment of bladder cancer, non-invasive bladder cancer, superficial bladder cancers (SBCs), transitional-cell carcinoma of the bladder, or a cancer that is TNM stage Ta or T1 and/or histologic grade G1 or G2.

Apaziquone (recommended INN) is also known as EO9 or NSC-382459. Chemically it is 5-(aziridin-1-yl)-3-(hydroxymethyl)-2-[(1*E*)-3-hydroxyprop-1-enyl]-1-methyl-1*H*-indole-4,7-dione (INN) with the structural formula:

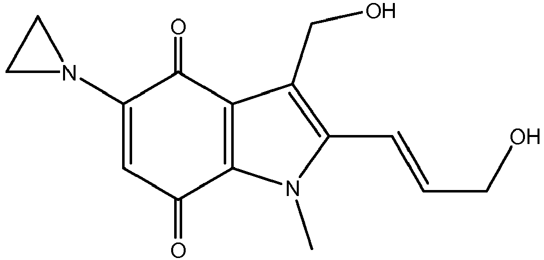

Apaziquone is a fully synthetic bioreductive alkylating indoloquinone. It is a pro-drug that generates cytotoxic species after enzymatic activation. The enzyme DTD (DT-diaphorase, also called NAD(P)H:quinone oxidoreductase-1, or NQO1) plays a prominent role in the activation of apaziquone under aerobic conditions. Apaziquone is also cytotoxic under hypoxic conditions, such as in cells with low DTD activity. The basic mechanism of activation of apaziquone is believed to be similar to that of other indoloquinones, involving reduction by cellular enzymes that transfer one or two electrons, forming semiquinone and hydroquinone, respectively. Oxidation of the semiquinone under aerobic conditions results in a redox cycle that can cause cell death by forming reactive oxygen species (ROS), resulting in DNA strand breaks. The semiquinone / hydroquinone can, particularly under hypoxic conditions, alkylate and crosslink DNA and other macromolecules, causing cell death.

The reductases expressed in tumors may play an important role in the selectivity of apaziquone. NQO1 (NAD(P)H: quinone oxidoreductase), a two electron reductase enzyme, may selectively target oxygenated cells, while one electron reducing enzymes such as Cytochrome P450 reductase may be more effective in targeting hypoxic cells. Loadman et al., 137 Br. J. Pharmacol. 701-709, 2002.

Lyophilized preparations of apaziquone have improved stability. Such preparations may include a bulking agent such as, but not limited to, maltitol, mannitol, xylitol, sorbitol, isomaltose, oligofructose and polydextrose. The lyophilized apaziquone preparation may also include a pH controlling agent in an amount sufficient to assist in maintaining pH at a near neutral range. The pH controlling agent may include, but is not limited to, sodium carbonate, potassium carbonate, calcium hydroxide, sodium hydroxide, magnesium hydroxide, potassium hydroxide, sodium bicarbonate, magnesium oxide or calcium oxide.

The lyophilized preparations of apaziquone as described herein may be reconstituted with any pharmaceutically acceptable diluent to prepare a pharmaceutically acceptable solution for administration to the patient after TUR. The pharmaceutical diluents useful for reconstituting the lyophilized preparations of the present disclosure may include, but are not limited to, diluents having propylene glycol, sodium bicarbonate, EDTA, and/or water. In accordance with the present invention the dosage route is by intravesical instillation. Dosage amounts may vary due to several factors including, but not limited to, individual patient characteristics, type and/or stage of cancer, or the specific formulation.

In previous Phase I and Phase II clinical trials with apaziquone as a cancer treatment, apaziquone was given intravenously. The drug was relatively well tolerated by the 129 patients treated by intravenous injection of apaziquone. Dose-limiting toxicity following intravenous administration was proteinuria. Despite reports of three partial responses in phase I studies, no responses were observed in phase II clinical trials when the drug was intravenously administered. The most likely explanation for the absence of tumor response is that apaziquone has a half-life of 0-19 minutes in the bloodstream and therefore, when intravenously administered, its rapid pharmacokinetic elimination effectively compromised drug delivery to tumors.

The pharmacokinetic properties of apaziquone that make it unfavorable for intravenous administration can be advantageous in the treatment of superficial cancers that arise in a readily accessible third compartment like the urinary bladder because intravesical administration circumvents the described problems of intravenous drug delivery. Retention of the drug within the bladder for one hour can improve drug penetration and the delivery of significant quantities into tumors while its absorption in the bloodstream remains unlikely. Even, however, if any drug reached the systemic circulation it would be rapidly cleared, minimizing the risk of systemic toxicity. Based on the foregoing and following studies confirming the presence of both elevated levels of DTD and regions of hypoxia in superficial bladder cancer, apaziquone was formulated as EOquin® for use in intravesical instillation in the treatment of SBCs.

Pharmaceutical compositions containing the active ingredient according to the present disclosure are suitable for administration to humans or other mammals. Typically, the pharmaceutical compositions are sterile, and contain no toxic, carcinogenic, or mutagenic compounds that would cause an adverse reaction when administered. Administration of the pharmaceutical composition can be performed before, during, or after the onset of solid tumor growth.

The "active ingredient" refers to the active moiety in the composition and includes apaziquone, or a physiologically acceptable salt, or solvate thereof. The active ingredients can be administered as the neat compound, or as a pharmaceutical composition containing one or more entities.

The "pharmaceutical composition" or "therapeutic composition" include those wherein the active ingredients are administered in an effective amount to achieve their intended purpose. More specifically, a "therapeutically effective amount" or "therapeutic amount" means an amount effective to prevent development of, to eliminate, to retard the progression of, or to reduce the size of a solid tumor. Determination of a therapeutically effective amount is well within the capability of those skilled in the art, especially in light of the detailed disclosure provided herein.

A "therapeutically effective dose" or "therapeutically effective" refers to that amount of the active ingredients that result in achieving the desired effect. Toxicity and therapeutic efficacy of such active ingredients can be determined by standard pharmaceutical procedures in cell cultures or experimental animals, e.g., determining the LD50 (the dose lethal to 50% of the population) and the ED50 (the dose therapeutically effective in 50% of the population). The dose ratio between toxic and therapeutic effects is the therapeutic index, which is expressed as the ratio between LD50 and ED50. A high therapeutic index is preferred. The data obtained can be used in formulating a range of dosage for use in humans. The dosage of the active ingredients preferably lies within a range of circulating concentrations that include the ED50 with little or no toxicity. The dosage can vary within this range depending upon the dosage form employed, and the route of administration utilized.

The exact formulation and dosage may be determined by an individual physician in view of the patient's condition and the type or stage of cancer.

The amount of pharmaceutical composition administered may be dependent on the subject being treated, on the subject's weight, the severity of the affliction, the manner of administration, and the judgment of the prescribing physician.

The active ingredients may be administered alone, or in admixture with a pharmaceutical carrier selected with regard to the intended route of administration and standard pharmaceutical practice. Pharmaceutical compositions for use in accordance with the present disclosure thus may be formulated in a conventional manner using one or more physiologically acceptable carriers comprising excipients and auxiliaries that facilitate processing of the active ingredients into preparations which can be used pharmaceutically.

When a therapeutically effective amount of the active ingredients is administered, the composition may be in the form of a pyrogen-free, parenterally acceptable aqueous solution. The preparation of such parenterally acceptable solutions, having due regard to pH, isotonicity, stability, and the like, is within the skill in the art.

"Start of instillation" is the time instillation of the therapeutic composition begins, following TUR-BT, while the "End of retention" is the time the administered therapeutic composition and other bladder contents are drained or voided, i.e. the time at which retention of the drug in the bladder is terminated.

For veterinary use, the active ingredients are administered as a suitably acceptable formulation in accordance with normal veterinary practice. The veterinarian can readily determine the dosing regimen that is most appropriate for a particular animal.

The following Examples are provided as illustrative embodiments of the present disclosure. However, the present disclosure should not be limited in any way by the Examples set forth below. Any one or more features of any Example may be combined with any one or more feature of any other embodiment or Example of the presently described methods, without departing from the scope of the present disclosure.

### Apaziquone Intravesical Instillation Studies

For studies described in Examples 1 and 2, EOquin® was reconstituted with 20 mL of its diluent, and further diluted with 20 mL water for injection to a total instillate volume of 40 mL. It was instilled into the patients' urinary bladder through a urethral catheter, two weeks after transurethral resection of bladder tumor (TUR-BT). The instillate was retained in the bladder for one hour. A treatment course consisted of 6 instillations one week apart.

### Example 1 (not part of the invention).

In a first study using intravesical administration, EOquin® was given to 12 patients with SBC, TNM stages Ta or T1, histologic grade G1 or G2. All patients had recurrent, multiple (2 to 10) superficial tumors of which all but one "marker lesion", 0.5-1 cm in diameter, were excised by transurethral resection (TUR-BT) prior to the trial. Tumor response was defined as complete response (CR), no response (NR) or progressive disease (PD) confirmed by cystoscopy and histology (biopsy of the initial marker lesion site, or complete resection of any residual or new lesion) four weeks after the last instillation. EOquin® was given weekly for six weeks, starting two weeks after TUR-BT.

In the phase I part of the study, six patients were treated with EOquin® concentrations that were increased weekly by 100% (0.0125 mg/mL; 0.025 mg/mL; 0.05 mg/mL; 0.1 mg/mL; 0.2 mg/mL; and 0.4 mg/mL and in this particular example, 0.5, 1, 2, 4, 8 and 16 mg in 40 mL). Following the determination of a recommended dose in this intra-patient dose escalation cohort, six additional patients received treatment at a fixed weekly dose of 0.1 mg/mL (in this particular example, 4 mg EOquin® in 40 mL fluid). Two patients in the intra-patient dose escalation cohort experienced EOquin®-related local side effects after the 0.2 mg/mL dose and received 0.1 mg/mL at their 6th instillation. The recommended dose was 0.1 mg/mL (4 mg in 40 mL of instillation fluid).

**Efficacy.** Tumor response four weeks after the last instillation was 4 / 6 Complete Responses in the fixed-dose cohort. It also was 4 CR out of 6 in the dose-escalation cohort.

**Safety.** During the intra-patient dose escalation, mild or moderate bladder pain and dysuria were seen at all dose levels. Hematuria was seen only at the 0.2 mg/mL and 0.4 mg/mL dose levels. Other reported complaints (one case each) were nausea, tongue discoloration and hot flush at the 0.0125 mg/mL level and a vasovagal event at 0.2 mg/mL. Almost all adverse effects were grade 1 or 2. There were no deaths on the study.

### Example 2

This study was a multi-center, non-randomized, open-label phase II study in patients with primary or recurrent, multiple (2-10) lesions of Ta or T1, G1 or G2 transitional cell SBC. Patients had undergone TUR-BT of all but one (marker) lesion, 0.5-1 cm in diameter, before study entry.

Instillations were performed weekly for six weeks. Tumor response was confirmed by biopsy, or complete resection of any residual tumor, two to four weeks after the last instillation. Tumor response was assessed as complete response (CR), no response (NR) or progressive disease (PD).

Of 46 patients entered, 37 had recurrent SBC. More than 50% of the patients had prior TUR-BT plus intravesical immunotherapy and/or chemotherapy. In total 17% had undergone TUR-BT alone and 17% had received no prior treatment. Tumor grade was reclassified as G3 at histology review in two patients.

**Efficacy.** Tumor response was 31 CR in 46 patients (67%). There were no cases of "progressive disease", i.e., progression to a T grade higher than 1 at the time of response evaluation, as defined in the protocol.

**Safety.** Treatment was well tolerated by the majority of the patients and there were few systemic (not bladder-related) adverse effects that were considered EOquin®-related.

The activity of EOquin® intravesical instillation compares favorably to that of other cytotoxic or immunologic agents studied in marker lesions of superficial bladder cancer. Safety data in a total of 58 patients in the Phase I and Phase II studies also compares well to that of the other chemotherapeutic agents currently used in intravesical instillation. Systemic side effects were reported infrequently. The total intravesical instillation dose of 4 mg corresponds to 20% or less of the tolerable weekly i.v. dose (12 mg/m² equivalent to a total dose of 20 mg in a patient with 1.7 m² body surface area) was given to 111 patients. In a pharmacokinetic study performed by HPLC analysis during the course of the first (phase I/II) study of intravesical administration, no detectable levels of apaziquone or its main metabolite were found in the plasma.

### Example 3.

Traditionally, adjuvant instillation treatment was started approximately 2 weeks after TUR-BT. The practice of a single instillation given within the 6 hours (from the time when TUR-BT is performed to about 6 hours after TUR-BT) following TUR-BT is more recent. "Immediate" (within 6 hours) or same-day post-TURBT intravesical instillation of cytotoxic agents was employed in Europe without reports of excessive toxicity when compared to those receiving treatment 2 or more weeks following TURBT. As Examples 1 and 2 demonstrate, EOquin® as a single agent is safe and generally well tolerated at a dose of 0.1 mg/mL (here, 4 mg in 40 mL), given weekly for 6 consecutive weeks in patients who have undergone TUR-BT, starting approximately 2 weeks after TUR-BT.

This study assessed the tolerability and safety of administering EOquin® immediately (i.g. within about 6 hours) following TUR-BT in patients with superficial bladder cancer. Plasma levels were measured in patients at selected sites to assess the degree of systemic absorption of EOquin® following intravesical instillation immediately (i.e. within about 6 hours) following TUR-BT.

Patient inclusion criteria included: (1) Transitional-cell carcinoma of the bladder, clinical TNM stage Ta or T1 and histologic grade G1 or G2 before transurethral resection; (2) Absolute neutrophil count ≥ 1.5 x 10⁹/L, platelets ≥ 100 x 10⁹/L, serum creatinine and bilirubin ≤ 1.5 x upper limits of local norm (ULN), serum GOT and GPT (AST / ALT) ≤ 3 x ULN; (3) Negative pregnancy test results in women of child-bearing age; (4) Agreement by all patients to use an effective method of contraception; (5) Eastern Cooperative Oncology Group (ECOG) performance status of 0-2 (ECOG scale: 0: Fully active, able to carry on all pre-disease performance without restriction; 1: Restricted in physically strenuous activity but ambulatory and able to carry out work of a light or sedentary nature, e.g., light house work, office work; 2: Ambulatory and capable of all self care but unable to carry out any work activities. Up and about more than 50% of waking hours; 3: Capable of only limited self-care, confined to bed or chair more than 50% of waking hours; and 4: Completely disabled. Cannot carry on any self-care. Totally confined to bed or chair); (6) Age ≥ 18; and (7) Patient fully informed of the investigational nature of the study; signed written informed consent.

Following TUR-BT, after ensuring complete hemostasis and absence of bladder perforation, patients received 0.1 mg/mL (4 mg apaziquone in 40 mL of instillate). EOquin® was given intravesically within 6 hours, of completion of TUR-BT and retained for 1 hour. Transurethral resection included resection of all visible tumors, reaching into the lamina propria, and biopsy of all suspect sites. The resected and biopsied material, which contained muscle to be evaluable for tumor depth, was preserved according to the standard procedures. The pathologists examined the tumor for histological grade and tumor stage. The operation summary, including size and location of tumors, were entered in the appropriate CRF.

Following TUR-BT, patients had an indwelling urethral catheter. The drug solution was instilled into the bladder by gravity drainage, or injected slowly using a catheter-tip syringe pre-filled with the prepared drug. The catheter was clamped once the drug has been delivered. Effort was made to avoid instillation of air bubbles into the bladder.

EOquin® was retained in the bladder for one (1) hour. Patients were monitored during the retention time and for 1 hour following drainage (total time 2 hours) for the development of local and systemic toxicities. The bladder was drained to an appropriate container at the end of the retention time.

Vital signs (blood pressure, heart rate and respiratory rate) were be recorded prior to the instillation, 30 minutes after the start of instillation and following the end of retention.

Pharmacokinetic investigations were performed in patients at selected sites who had given informed consent to blood sampling for these studies. The appropriate volume of blood (generally 1.5 ml) was drawn from the cannula or catheter, collected in heparinized tubes and immediately placed on ice. The sampling schedule was as follows:
- Sample 1:: Before the start of instillation of EOquin®;
- Sample 2:: 5 minutes after the start of instillation;
- Sample 3:: 15 minutes after the start of instillation;
- Sample 4:: 30 minutes after the start of instillation;
- Sample 5:: 45 minutes after the start of instillation; and
- Sample 6:: 60 minutes after the start of instillation (corresponding to the time of drainage of EOquin® as per protocol).

As soon as possible after collection, the blood was centrifuged at 4000 rpm for 5 min and the plasma transferred to 2 clean tubes for immediate storage at - 70 to -80 °C.

The primary endpoint of the study was determined by 2 main assessments: (1) Presence, severity and frequency of adverse events / toxicities in the 2 weeks following the instillation; and (2) For the first 10 patients with low-risk histology (stage Ta-T1, Grade G1-G2), cystoscopic evaluation of bladder epithelium approximately 3 months (± 2 weeks) after immediate post-TUR-BT instillation

Patients will be initially enrolled in the study based on the Investigator's visual assessment of the stage and grade of the tumor. Clinical assessment of the probable tumor stage and grade and adequacy of the resection was confirmed only later by histopathologic examination of the resection specimen. As a result, all patients enrolled in the study received an immediate post-TUR-BT instillation of EOquin®.

The following measures were taken between Days 6 and 12 (counting from the day of TUR-BT and hereinafter referred to as "Day 8"): (1) Physical examination (including assessment of performance status); (2) Recording of vital signs (blood pressure, pulse, temperature), weight; (3) Hematology: Hemoglobin, platelet count, WBC and differential count; (4) Serum chemistry: Serum creatinine, urea or BUN, sodium, potassium, calcium, albumin, SGOT / SGPT (AST/ALT); blood sugar; (5) Urinalysis: Macroscopic examination with pH, specific gravity, glucose, protein, nitrites blood. Microscopic examination noting WBC, RBC, casts, other; and (6) Recording of concomitant medications and adverse events.

The following measures were taken between days 13 and 17 (counting from the day of TUR-BT and hereinafter referred to as "Day 15"): (1) Physical examination (including assessment of performance status); (2) Recording of vital signs (blood pressure, pulse, temperature), weight; (3) Hematology: Hemoglobin, platelet count, WBC and differential count; (4) Serum chemistry: Serum creatinine, urea or BUN, sodium, potassium, calcium, albumin, SGOT / SGPT (AST/ALT)and blood sugar; (5) Urinalysis: Macroscopic examination, pH, specific gravity, glucose, protein, nitrites and blood; microscopic examination noting WBC, RBC, casts, other; and (6) Recording of concomitant medications and adverse events.

At the cystoscopy scheduled 3 months after TUR-BT, the Investigator assessed the state of the bladder mucosa in addition to screening for possible tumor recurrence. Observations on wounds, scarring and other bladder lesions were recorded. Biopsies were taken at the discretion of the Investigator. The following measurements were also taken within ± 2 weeks of the end of the third month following TUR-BT: (1) Physical examination; (2) Recording of vital signs (blood pressure, pulse, temperature), weight; (3) Hematology: Hemoglobin, platelet count, WBC and differential count; (4) Serum chemistry: Serum creatinine, urea or BUN, sodium, potassium, calcium, albumin, SGOT / SGPT (AST/ALT) and blood sugar; (5) Urinalysis: Macroscopic examination, pH, specific gravity, glucose, protein, nitrites and blood; microscopic examination noting WBC, RBC, casts, other; (6) Urine cytology; (7) Record of concomitant medications and adverse events (prior to cystoscopy); and (8) Follow-up cystoscopy, noting the findings on the Cystoscopy CRF, with urine cytology, and photograph if available. Patients with unexpected or abnormal findings at the cystoscopy performed at the end of the third month following TUR-BT were followed until the abnormality either resolved or stabilized.

The following table summarizes various activities and measures and the time point relative to TUR-BT at which they were taken:

| **Examination, Procedure or Form** | **Screening** (≤ 2 weeks before TUR-BT) | **Day on Study** | | | |
|---|---|---|---|---|---|
| | | **1** | **8** | **15** | **Mo 3²** |
| Medical History | **X** | | | | |
| Physical Exam | **X** | **X** | **X** | **X** | **X** |
| Vital Signs | **X³** | **X** | **X** | **X** | **X** |
| Hematology | **X** | **X** | **X** | **X** | **X** |
| Blood chemistry | **X** | **X** | **X** | **X** | **X** |
| Urinalysis | **X** | **X** | **X** | **X** | **X** |
| Urine cytology | **X** | | | | **X** |
| Pregnancy test (female patients) | **X** | | | | |
| TUR | | **X** | | | |
| EOquin™ instillation | | **X** | | | |
| Histologic examination | | **X** | | | |
| Blood Samples for pharmacokinetics | | **X¹** | | | |
| Cystoscopy | | | | | **X** |
| | | | | | |
| Adverse events | | **X** | **X** | **X** | **X** |
| Concomitant Meds | **X** | **X** | **X** | **X** | **X** |

This was a multicenter, single dose, safety and tolerability study. 20 patients were dosed with 4 mg of apaziquone. The dose was administered intravesically within 6 hours of TURBT. The dose was safe and well tolerated in most patients. The 3-month post-treatment cystoscopy showed reepithelialization at the resection site. Additionally, pharmacokinetic data detected neither apaziquone nor metabolites in the plasma sample following TUR-BT and drainage of instillate. Adverse events included genitourinary symptoms including dysuria, hematuria, urinary retention and frequency, similar to those seen following TURBT. No deaths were reported.

### Example 4.

In contrast to mitomycin (MMC), EOquin® is not a skin irritant and is not absorbed through the bladder mucosa when given intravesically (molecular weight 288). EOquin® has previously demonstrated activity against superficial bladder cancer in previous studies. The next described study evaluated the tolerability and safety of EOquin®, as well as its effect on surgical wound healing and systemic absorption when given intravesically as a single dose immediately after TURBT (Transurethral Resection of Bladder Tumor) in patients with superficial bladder cancer.

This was a multicenter, single-arm, open-label, safety study. Patients (N= 22) with ≤ 4 tumors with a maximal diameter of 3.5 cm, clinically stage Ta-T1 and grade G1-G2, received 4 mg of EOquin® in 40 mL instillate within 6 hours of TURBT. EOquin® was instilled via an indwelling Foley catheter that was then clamped for one hour. After an hour, the bladder was drained and the catheter was removed. Patients were assessed for adverse events during the one-hour retention and at follow-up visits on postoperative days 8 and 15. Wound healing was assessed by cystoscopy performed at postoperative day 85. Plasma samples for drug level assays were obtained from six patients at six time points: before instillation, and at 5, 15, 30, 45 and 60 minutes of instillation. The samples were analyzed by a fully validated method by using high performance liquid chromatography with tandem mass spectrometry (HPLC-MS/MS). The lower limit of quantitation (LLOQ) for EOquin® was 5 mg/mL and for its metabolite, EO5a, the LLOQ was 10 ng/mL.

23 patients were enrolled and 20 patients were dosed. Reasons for not dosing were: syncopal episode on the day prior to TURBT in one patient and the absence of tumor in two patients. EOquin® instillation and retention was well tolerated by all patients. No deaths or dropouts were reported. Four severe adverse events were reported in three patients: hematuria (x 2), cystitis and urinary retention. Day 85 cystoscopy showed reepithelialization without evidence of impaired wound samples.

In sum, a single, intravesical dose of EOquin® was well tolerated and safe when administered to patients immediately following TURBT for superficial (noninvasive) bladder cancer. EOquin was not absorbed in the bloodstream from the bladder mucosa.

Various adaptations and modifications of the embodiments can be made and used without departing from the scope of the present disclosure which can be practiced other than as specifically described herein. The above description is intended to be illustrative, and not restrictive. The scope of the presently described disclosure is to be determined only by the claims.

Any one or more features of any embodiment of the presently described methods can be combined with any one or more other features of any other embodiment of the present disclosure, without departing from the scope of the present disclosure.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Notwithstanding that
the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements.

The terms "a" and "an" and "the" and similar referents used in the context of describing the described methods (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the disclosure and does not pose a limitation on the scope of the present disclosure otherwise claimed. No language in the specification should be construed as indicating any non-claimed element essential to the practice of the present disclosure.

Groupings of alternative elements of the presently described methods are not to be construed as limitations. Each group member may be referred to and claimed individually or in any combination with other members of the group or other elements found herein, without departing from the scope of the present disclosure.

Any combination of the above- described elements in all possible variations thereof is encompassed by the methods described herein unless otherwise indicated herein or otherwise clearly contradicted by context.

Furthermore, numerous references have been made to patents and printed publications throughout this specification.

## Claims

1. Use of apaziquone (EO9) in the manufacture of a medicament for treating bladder cancers comprising administering via intravesical instillation a therapeutic composition comprising a therapeutic amount of apaziquone (EO9), wherein said administering occurs within about 6 hours following transurethral resection of bladder tumor (TUR-BT).

2. The use according to claim 1 wherein said administering occurs within about 5 hours following TUR-BT, more preferably within about 4 hours following TUR-BT, and most preferably within about 3 hours following TUR-BT.

3. The use according to claim 1, wherein said cancer is non-invasive bladder cancer, transitional-cell carcinoma of the bladder, TNM stage Ta or T1, or histologic grade G1 or G2.

4. The use according to claim 1, wherein said therapeutic composition comprises from about 1 mg to about 8 mg per dose apaziquone, preferably from about 2 mg to about 6 mg per dose apaziquone and more preferably from about 3 mg to about 5 mg per dose apaziquone.

5. Use of apaziquone (EO9) in the manufacture of a medicament for treating bladder cancers in a patient that has undergone transurethral resection of bladder tumor (TUR-BT) comprising the steps of:
a) providing a therapeutically effective amount of a reconstituted lyophilized therapeutic composition comprising from about 1 mg to about 8 mg per dose apaziquone; and
b) administering via intravesical instillation said reconstituted lyophilized therapeutic composition, after said TUR-BT, wherein said administering occurs within about 6 hours following TUR-BT.

6. The use according to claim 5, wherein said administering occurs within about 5 hours following TUR-BT, more preferably within about 4 hours following TUR-BT, and most preferably within about 3 hours following TUR-BT.

7. Apaziquone (EO9) for use in treating bladder cancers by administering via intravesical instillation a therapeutic composition comprising a therapeutic amount of apaziquone (EO9), wherein said administering occurs within about 6 hours following transurethral resection of bladder tumor (TUR-BT).

8. Apaziquone (EO9) for use according to claim 7 wherein said administering occurs within about 5 hours following TUR-BT, more preferably within about 4 hours following TUR-BT, and most preferably within about 3 hours following TUR-BT.

9. Apaziquone (EO9) for use according to claim 8, wherein said cancer is non-invasive bladder cancer, transitional-cell carcinoma of the bladder, TNM stage Ta or T1, or histological grade G1 or G2.

10. Apaziquone (EO9) for use according to claim 7, wherein said therapeutic composition comprises from about 1 mg to about 8 mg per dose apaziquone, preferably from about 2 mg to about 6 mg per dose apaziquone, and more preferably from about 3 mg to about 5 mg per dose apaziquone.

11. Apaziquone (EO9) for use in treating bladder cancers in a patient that has undergone transurethral resection of bladder tumor (TUR-BT) comprising the steps of:
a) providing a therapeutically effective amount of a reconstituted lyophilized therapeutic composition comprising from about 1 mg to about 8 mg per dose apaziquone; and
b) administering via intravesical instillation said reconstituted lyophilized therapeutic composition, after said TUR-BT, wherein said administrating occurs within about 6 hours following TUR-BT.

12. Apaziquone for use according to claim 11, wherein said administering occurs within about 5 hours following TUR-BT, more preferably within about 4 hours following TUR-BT, and most preferably within about 3 hours following TUR-BT.

## Patentansprüche

1. Verwendung von Apaziquon (EO9) bei der Herstellung eines Medikaments zur Behandlung von Blasenkrebs, umfassend die Verabreichung einer therapeutischen Zusammensetzung, die eine therapeutische Menge von Apaziquon (EO9) umfasst, durch intravesikale Instillation, wobei die Verabreichung innerhalb von etwa 6 Stunden nach der transurethralen Resektion des Blasentumors (TUR-BT) erfolgt.

2. Verwendung nach Anspruch 1, wobei die Verabreichung innerhalb von etwa 5 Stunden nach TUR-BT, stärker bevorzugt innerhalb von etwa 4 Stunden nach TUR-BT und am stärksten bevorzugt innerhalb von etwa 3 Stunden nach TUR-BT erfolgt.

3. Verwendung nach Anspruch 1, wobei der Krebs nicht-invasiver Blasenkrebs, Übergangszellkarzinom der Blase, TNM-Stadium Ta oder T1 oder histologischer Grad G1 oder G2 ist.

4. Verwendung nach Anspruch 1, wobei die therapeutische Zusammensetzung von etwa 1 mg bis etwa 8 mg pro Dosis Apaziquon, vorzugsweise von etwa 2 mg bis etwa 6 mg pro Dosis Apaziquon und stärker bevorzugt von etwa 3 mg bis etwa 5 mg pro Dosis Apaziquon umfasst.

5. Verwendung von Apaziquon (EO9) bei der Herstellung eines Medikaments zur Behandlung von Blasenkrebs bei einem Patienten, der eine transurethrale Resektion des Blasentumors (TUR-BT) durchlaufen hat, umfassend die Schritte von:
a) Bereitstellen einer therapeutisch wirksamen Menge einer rekonstituierten lyophilisierten therapeutischen Zusammensetzung, umfassend von etwa 1 mg bis etwa 8 mg pro Dosis Apaziquon; und
b) Verabreichung der rekonstituierten lyophilisierten therapeutischen Zusammensetzung durch intravesikale Instillation nach dem TUR-BT, wobei die Verabreichung innerhalb von etwa 6 Stunden nach dem TUR-BT erfolgt.

6. Verwendung nach Anspruch 5, wobei die Verabreichung innerhalb von etwa 5 Stunden nach TUR-BT, vorzugsweise innerhalb von etwa 4 Stunden nach TUR-BT und stärksten bevorzugt innerhalb von etwa 3 Stunden nach TUR-BT erfolgt.

7. Apaziquon (EO9) zur Verwendung bei der Behandlung von Blasenkrebs durch Verabreichung einer therapeutischen Zusammensetzung durch intravesikale Instillation, umfassend eine therapeutische Menge von Apaziquon (E09), wobei die Verabreichung innerhalb von etwa 6 Stunden nach der transurethralen Resektion des Blasentumors (TUR-BT) erfolgt.

8. Apaziquone (EO9) zur Verwendung nach Anspruch 7, wobei die Verabreichung innerhalb von etwa 5 Stunden nach TUR-BT, vorzugsweise innerhalb von etwa 4 Stunden nach TUR-BT und am stärksten bevorzugt innerhalb von etwa 3 Stunden nach TUR-BT erfolgt.

9. Apaziquone (EO9) zur Verwendung nach Anspruch 8, wobei der Krebs nicht-invasiver Blasenkrebs, Übergangszellkarzinom der Blase, TNM-Stadium Ta oder T1 oder histologischer Grad G1 oder G2 ist.

10. Apaziquon (EO9) zur Verwendung nach Anspruch 7, wobei die therapeutische Zusammensetzung von etwa 1 mg bis etwa 8 mg pro Dosis Apaziquon, vorzugsweise von etwa 2 mg bis etwa 6 mg pro Dosis Apaziquon, und insbesondere von etwa 3 mg bis etwa 5 mg pro Dosis Apaziquon umfasst.

11. Apaziquone (EO9) zur Verwendung bei der Behandlung von Blasenkrebs bei einem Patienten, der einer transurethralen Resektion des Blasentumors (TUR-BT) unterzogen wurde, umfassend die Schritte von:
a) Bereitstellen einer therapeutisch wirksamen Menge einer rekonstituierten lyophilisierten therapeutischen Zusammensetzung, umfassend von etwa 1 mg bis etwa 8 mg pro Dosis Apaziquon; und
b) Verabreichen der rekonstituierten lyophilisierten therapeutischen Zusammensetzung durch intravesikale Instillation nach dem TUR-BT, wobei die Verabreichung innerhalb von etwa 6 Stunden nach dem TUR-BT erfolgt.

12. Apaziquon zur Verwendung nach Anspruch 11, wobei die Verabreichung innerhalb von etwa 5 Stunden nach TUR-BT, vorzugsweise innerhalb von etwa 4 Stunden nach TUR-BT und am stärksten bevorzugt innerhalb von etwa 3 Stunden nach TUR-BT erfolgt.

## Revendications

1. Utilisation d'apaziquone (EO9) dans la fabrication d'un médicament pour le traitement de cancers de la vessie comprenant l'administration, par l'intermédiaire d'une instillation intravésicale, d'une composition thérapeutique comprenant une quantité thérapeutique d'apaziquone (EO9), ladite administration intervenant dans les 6 heures environ qui suivent la résection transuréthrale de la tumeur à la vessie (TUR-BT).

2. Utilisation selon la revendication 1, dans laquelle ladite administration intervient dans les 5 heures environ qui suivent la TUR-BT, de manière davantage préférée dans les 4 heures environ qui suivent la TUR-BT, et de manière préférée entre toutes dans les 3 heures environ qui suivent la TUR-BT.

3. Utilisation selon la revendication 1, dans laquelle ledit cancer est un cancer non invasif de la vessie, un carcinome à cellules transitoires de la vessie, un stade TNM Ta ou T1, ou un grade histologique G1 ou G2.

4. Utilisation selon la revendication 1, dans laquelle ladite composition thérapeutique comprend entre environ 1 mg et environ 8 mg par dose d'apaziquone, de préférence entre environ 2 mg et environ 6 mg par dose d'apaziquone et de manière davantage préférée entre environ 3 mg et environ 5 mg par dose d'apaziquone.

5. Utilisation d'apaziquone (EO9) dans la fabrication d'un médicament pour le traitement de cancers de la vessie chez un patient ayant subi une résection transuréthrale de la tumeur à la vessie (TUR-BT) comprenant les étapes :
a) de fourniture d'une quantité thérapeutiquement efficace d'une composition thérapeutique lyophilisée reconstituée comprenant entre environ 1 mg et environ 8 mg par dose d'apaziquone ; et
b) d'administration, par l'intermédiaire d'une instillation intravésicale, de ladite composition thérapeutique lyophilisée reconstituée, après ladite TUR-BT, ladite administration intervenant dans les 6 heures environ qui suivent la TUR-BT.

6. Utilisation selon la revendication 5, dans laquelle ladite administration intervient dans les 5 heures environ qui suivent la TUR-BT, de manière davantage préférée dans les 4 heures environ qui suivent la TUR-BT, et de manière préférée entre toutes dans les 3 heures environ qui suivent la TUR-BT.

7. Apaziquone (EO9) destinée à être utilisée dans le traitement de cancers de la vessie par l'administration, par l'intermédiaire d'une instillation intravésicale, d'une composition thérapeutique comprenant une quantité thérapeutique d'apaziquone (EO9), ladite administration intervenant dans les 6 heures environ qui suivent la résection transuréthrale de la tumeur à la vessie (TUR-BT).

8. Apaziquone (EO9) destinée à être utilisée selon la revendication 7, dans laquelle ladite administration intervient dans les 5 heures environ qui suivent la TUR-BT, de manière davantage préférée dans les 4 heures environ qui suivent la TUR-BT, de manière préférée entre toutes dans les 3 heures environ qui suivent la TUR-BT.

9. Apaziquone (EO9) destinée à être utilisée selon la revendication 8, dans laquelle ledit cancer est un cancer non invasif de la vessie, un carcinome à cellules transitoires de la vessie, un stade TNM Ta ou T1, ou un grade histologique G1 ou G2.

10. Apaziquone (EO9) destinée à être utilisée selon la revendication 7, dans laquelle ladite composition thérapeutique comprend entre environ 1 mg et environ 8 mg par dose d'apaziquone, de préférence entre environ 2 mg et environ 6 mg par dose d'apaziquone et de manière davantage préférée entre environ 3 mg et environ 5 mg par dose d'apaziquone.

11. Apaziquone (EO9) destinée à être utilisée dans le traitement de cancers de la vessie chez un patient qui a subi une résection transuréthrale de la tumeur à la vessie (TUR-BT) comprenant les étapes :
a) de fourniture d'une quantité thérapeutiquement efficace d'une composition thérapeutique lyophilisée reconstituée comprenant entre environ 1 mg et environ 8 mg par dose d'apaziquone ; et
b) d'administration, par l'intermédiaire d'une instillation intravésicale, de ladite composition thérapeutique lyophilisée reconstituée, après ladite TUR-BT, ladite administration intervenant dans les 6 heures environ qui suivent la TUR-BT.

12. Apaziquone destinée à être utilisée selon la revendication 11, dans laquelle ladite administration intervient dans les 5 heures environ qui suivent la TUR-BT, de manière davantage préférée dans les 4 heures environ qui suivent la TUR-BT, et de manière préférée entre toutes dans les 3 heures environ qui suivent la TUR-BT.
